(19) **Europäisches Patentamt**
**European Patent Office**
**Office européen des brevets**

(11) **EP 4 481 758 A1**

(12) **EUROPEAN PATENT APPLICATION**

(43) Date of publication:
**25.12.2024 Bulletin 2024/52**

(21) Application number: **23180247.1**

(22) Date of filing: **20.06.2023**

(51) International Patent Classification (IPC):
**G16H 40/20** (2018.01)    **G16H 50/70** (2018.01)
**G16H 10/60** (2018.01)    **G16H 20/00** (2018.01)
**G16H 30/20** (2018.01)

(52) Cooperative Patent Classification (CPC):
**G16H 30/20; G16H 10/60; G16H 20/00;**
**G16H 40/20; G16H 50/70;** G16H 40/63;
G16H 40/67

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **SCHADEWALDT, Nicole**
**Eindhoven (NL)**
• **BULUT, Murtaza**
**Eindhoven (NL)**
• **SAINI, Privender Kaur**
**5656AG Eindhoven (NL)**
• **KLAASSEN, Remy**
**Eindhoven (NL)**
• **VAN EE, Raymond**
**Eindhoven (NL)**

(74) Representative: **Philips Intellectual Property &**
**Standards**
**High Tech Campus 52**
**5656 AG Eindhoven (NL)**

(54) **TIME CONTROLLED ACQUISITION OF MEDICAL DATA**

(57)    Disclosed herein is a method comprising: receiving a user profile indicating an initial state of the user, wherein the duration of an acquisition process from the user in the initial state is an acquisition time period; determining a timeline representing a chronological sequence of interactive programs and associated time intervals; selecting user systems that are configured to perform the interactive programs, using the locations of the user systems to determine a route from a current location of the user to the location of the medical system; providing the route and the timeline for guidance of the user through the route, the providing comprising controlling the selected user systems to execute the interactive programs in accordance with the time intervals of the timeline; thereby changing the initial state of the user; and thereafter initiating the acquisition process from the user for a reduced acquisition time period.

Fig. 1

**EP 4 481 758 A1**

**Description**

FIELD OF THE INVENTION

The invention relates to medical acquisition systems, in particular to a method for acquisition of image data.

BACKGROUND OF THE INVENTION

The acquisition of medical imaging data such as magnetic resonance imaging (MRI) data is usually performed on a limited number of patients per day. The patient may have to wait for several months in order to be imaged. However, this is not only due to the limited number of acquisition systems but also due to the duration of the acquisition process which may be too long and not accurately predictable. This renders the task of reducing the idle time of acquisition systems and increasing the number of acquisitions per single acquisition system a real technical challenge.

SUMMARY OF THE INVENTION

The invention provides for a medical system, a computer program, and a method in the independent claims. Embodiments are given in the dependent claims.

In one aspect the invention provides for a method for acquisition of medical imaging data using an acquisition process from a user, wherein a duration of the acquisition process depends on a state of the user to be imaged. The method comprises:

- providing a medical system and multiple user systems, the systems being distributed according to predefined locations;
- receiving a user profile indicating an initial state of the user, wherein the duration of the acquisition process from the user in the initial state is an acquisition time period;
- determining an acquisition scheme comprising:

  - determining a timeline representing a chronological sequence of interactive programs and associated time intervals, the interactive programs being configured to change the initial state of the user;
  - selecting user systems that are configured to perform the interactive programs,
  - using the locations of the user systems to determine a route from a current location of the user to the location of the medical system;

- providing the route and the timeline for enabling guidance of the user through the route, thereby changing the initial state of the user, the providing comprising controlling the selected user systems to execute the interactive programs in accordance with the time intervals of the timeline; and thereafter

- initiating the acquisition process from the user for reducing the acquisition time period.

The medical imaging data (also referred to as medical data) may be MRI data, wherein the medical system may be an MRI system. Alternatively, the medical data may be computed tomography (CT) data, wherein the medical system may be a CT system. Alternatively, the medical data may be image guided therapy (IGT) data, wherein the medical system may be a IGT system. Alternatively, the medical data may be diagnostic X-ray (DXR) data, wherein the medical system may be a DXR system. The acquisition of the medical imaging data from the user may be performed using an acquisition process. The acquisition process may include a preparation phase and an acquisition phase. The acquisition phase may comprise using the medical system to acquire the medical data from the user. The user may, for example, be a patient. The preparation phase may be used for training the user. The training of the user may comprise multiple training steps. The training steps may be required for enabling acquisition of data. The acquisition time period may be the time $t_{proc}^{s_0}$ required to complete the acquisition process. The time $t_{proc}^{s_0}$ required to complete the acquisition process may be the time ( $t_{prep}^{s_0}$ ) required for the preparation phase in addition to the time required for the acquisition phase ( $t_{acq}^{s_0}$ ). That is, $t_{proc}^{s_0} = t_{prep}^{s_0} + t_{acq}^{s_0}$, where the superscript $s_0$ refers to a (initial) state of the user, and the subscripts *proc*, *prep* and *αcq* refer to the acquisition process, the preparation phase and the acquisition phase respectively. The time $t_{prep}^{s_0}$ of the preparation phase may be the sum of the times required by the training steps. For example, if the number of training steps is *N*, the time of the preparation phase may be the sum of the training times: $t_{prep}^{s_0} = \sum_{i=1}^{N} t_{train}^{s_0,i}$. For example, the first training step may last the time $t_{train}^{s_0,1}$, the second training step may last the time $t_{train}^{s_0,2}$ and so forth. The time $t_{proc}^{s_0}$ required to complete the acquisition process may, for example, be an expected time or may be estimated from previous acquisition processes of the same user or of other users which are performed without using the present method.

The state of the user may refer to at least one of: training status of the user, one or more vital signs of the user, functional status of the user, psychological state of the user or physiological state of the user. The functional status of the user may, for example, be defined by the posture of the user and/or gait of the user. The training status may be defined by the number of training steps followed or attended by the user and/or the time spent by the user in one or more training steps. The physiological state of the user may be defined by evaluating a physiological state metric. The physiological state metric may be defined using (or derived from) at least one of: eye and eyelids movements of the user, pupil size of the user, speech or sounds generated by the user, facial expressions of the user, or brainwave energy and frequencies of the user. The psychological state of the user may refer to the affective state of the user such as the emotion, mood, and attitudes of the user. Emotions may be shorter-term and last from seconds to minutes. Moods may be longer-term lasting for hours. Attitudes may be longer term, reflecting the general behavior and life philosophy. The psychological state of the user may be defined by evaluating a psychological state metric. The psychological state metric may be defined using (or derived from) at least one of: heart rate of the user, heart rate variability of the user, respiration rate of the user, skin conductance of the user, speech signal features of the user, speech content features of the user, facial expressions of the user, eye movements of the user, body part movements of the user, body posture of the user, gait patterns of the user, or interaction patterns of the user. The conversational characteristics of the user may comprise length of utterances, silence amount and durations, number of interactions, and type of sentences. The interaction patterns may include user-interface features such as selected characters, selected colors, sounds/music, the speed of typing, the pressure of pressing, number of typing errors, frequency of errors.

The psychological state metric may, for example, comprise at least one of: valence, Arousal or motivational intensity. The valence refers to how positive or negative the emotion is, and can have a value in a scale e.g., from -100 to 100. The Arousal refers to the level of activation (of the sympathetic nervous system) of the user. It may also have a value in the scale -100 to 100. The dominance or motivational intensity may correspond to the impulsion to act. The motivational intensity may also have a value on the scale from -100 to 100. The psychological state metric may be evaluated by computing the valence and/or Arousal and/or motivational intensity. This may enable to define the psychological state metric in a multi-dimensional space defined by the valence, Arousal and motivational intensity. In another example, the psychological state metric may be evaluated using physiological and behavioral parameters of the user to classify the psychological state of the user in one of the emotional categories such as anger, happiness, sadness and fear e.g., if the facial expression of the user indicates smile and speech pitch there is a high likelihood that the user is in happy state.

Embodiments may enable to train the patients before they even get to the exam waiting room. It may be based on detecting the patient's profile. Several experience zones within the hospital may be provided to help with different trainings. An artificial intelligence (AI) engine may, for example, determine a personalized patient route to the exam room to let him pass by experience zones to train him and at the same time manage the waiting time. Thus, the patient arrives at the exam room well trained and on time, ready to acquire medical data in an optimal manner.

In one example, the state of the user may comprise the training status of the user. Alternatively, or additionally, the state of the user may comprise the psychological state of the user. Alternatively, or additionally, the state of the user may comprise the physiological state of the user. Alternatively, or additionally, the state of the user may comprise the functional status of the user.

Changing the state of the user may comprise at least one of: changing the training status of the user, changing the one or more vital signs of the user, changing the psychological state of the user, changing the physiological state of the user or changing the functional status of the user. For example, the initial state $s_0$ of the user may indicate that the number of training steps of the user is zero. Changing the initial state $s_0$ may thus result in the number of training steps of the user which is higher than zero. The psychological state of the user may be changed, for example, by at least one of: sensory (e.g., Light, audio, image, olfactory, haptic) stimuli, interaction patterns (e.g., by asking the user to interact in certain way with certain characters), or by physical movement.

The time $t_{proc}^{s_0}$ required to complete the acquisition process may depend on the state $s_0$ of the user. For example, if the training status of the user is below a threshold, or the vital sign is not in a target range, or the physiological state and psychological states are not in respective target range, the acquisition process may take longer time. The time $t_{proc}^{s_0}$ required to complete the acquisition process may thus be too long if the state of the user is not in a desired value. In addition, the prediction of the duration of the acquisition process may be highly inaccurate. This may also render the prediction of the start time of the acquisition process inaccurate. However, in order to perform a time efficient acquisition process, there may be a need of a short process time and a more accurate prediction of the time of the acquisition process. For example, the shorter the acquisition process time, the more accurate the estimation of the time window during which the user would be imaged. This may enable to acquire image data from a higher number of users with an optimal time scheduling because the acquisition time period may be shorter and the start time of the acquisition process may be accurately predicted for each user. This may reduce the idle time of the acquisition system.

For example, the acquisition of the medical data from the user using the medical system may be performed using the state of the user. For example, one or more attributes that indicate the current state of the user may be used to control the medical system to acquire the medical data from the user. E.g. the current state of the user may indicate heart related state parameters such as heart rate, respiration rate or psychological state which may be used to adapt or control the MRI system to acquire data for cardiac MR imaging.

The present subject matter may enable a proper operation of the medical system by a guided human-machine interaction process. The present subject matter may reduce the time $t_{proc}^{s_0}$ required to complete the acquisition process e.g., the acquisition process time $t_{proc}^{s_1}$ in the new state $s_1$ is smaller than the (expected or estimated) acquisition process time $t_{proc}^{s_0}$ in the initial state $s_0$. For example, the present subject matter may reduce the time $t_{prep}^{s_0}$ required for the preparation phase by reducing to zero or almost zero one or more training times $t_{train}^{s_0,i}$, e.g. the time for explanations needed by the staff might be reduced to zero, if sufficient preparation by user systems is provided. Indeed, after being guided through the route, the user may be in the state $s_1$ which is different from the initial state $s_0$. If a i-th training step is performed during the route it may be skipped during the acquisition process, and thus the corresponding training time $t_{train}^{s_0,i}$ may be reduced to zero, that is, $t_{train}^{s_1,i} = 0$. In addition, even if the i-th training step is not skipped during the acquisition process, the corresponding training time $t_{train}^{s_1,i}$ may be smaller than the (initial) training time $t_{train}^{s_0,i}$ because the user may have performed at least part of the training step and would thus require less time to compete the training step during the acquisition process. In addition, without following the training along the route according to the present subject matter, there may be a high likelihood that the acquisition fails and then repeated again.

In one example, the acquisition process may be initiated or started if the new state $s_1$ of the user that results from changing the state $s_0$ of the user has a target value. The target value may be a target training status and/or one or more target vital signs and/or target functional status and/or one target physiological state and/or one target psychological state. The target training status may, for example, be a minimum number of training steps. The target vital sign may be a target value or a target range of values of the vital sign (e.g., the heart rate is in a target range). The target functional status may, for example, be a specific value of the posture of the user and/or of the gait of the user. The target physiological state may be a desired value of the target physiological state metric. The target psychological state may be a desired value of the psychological state metric. Depending on the definition of the state of the user, the target value may be provided as a scalar or a vector or other data structures. For example, if the state of the user is the training status of the user, the target value may be a scalar indicating the number of training steps. If the state of the user is the training status and the psychological state of the user, the target value may be a vector with two entries, the first entry indicating the number of training steps and the second entry indicating a range or a value of the psychological state metric. If the new state of the user does not have the target value, the acquisition scheme may be adapted using the updated user profile, and the user may be guided through the updated route and interactive programs. If the user is in a relaxed state, some training steps may be skipped from the preparation phase, resulting is shorter acquisition times. This repetition of the update of the user profile, the update of the acquisition scheme and the guidance of the user may be performed until the state of the user has the target value.

In one example, the acquisition scheme may be determined using an expected user speed e.g., the time intervals may be defined using the current location of the user, the route and the expected speed of the user. The user speed may, for example, be estimated by the acquisition scheduler system using the user profile. In addition, the time interval of the same interactive program may be set differently depending on the user's age e.g., more time may be provided for children (e.g., children may be slower on some stations but faster on others).

The present subject matter may use interactive programs that enable to train the user in order to assist the user to be imaged in a time efficient manner. The interactive programs may be provided as part of the acquisition scheme that is determined by the present subject matter. The interactive program may be a computer program. The interactive program may be executed by the user system to which the computer program is assigned. The interactive program may use user interaction to operate. The user interaction may include at least one of: inputting information, modifying information, or a presence of the user in front of the user system that executes the interactive program. The user interaction may be performed by the patient. Additionally, the user interaction of one or more interactive programs may be performed by another user such as an administrator of the acquisition scheduler system.

In one example, the interactive program may provide instructions to the user for acquisition of the medical data. In one example, the interactive programs may provide guidelines or medical data acquisition information or experience regarding the conditions in which the medical data is acquired such as the noise level to be expected. For example, if the user is scheduled to receive an MR examination, the interactive program might provide the exact noise for the planned scan sequence over headphone, but shortened and at a reduced volume, to accustomize the user to the types of noises to be expected. The feedback of the user might be a control of the volume, such that the user can herself increase the volume until the expected volume is reached, such that she is prepared for what to expect. The provided medical data acquisition information or experience may enable to change one or more vital signs of the user such as the heart rate and may improve the information level or experience of the user. The medical data information may indicate the order of steps of the acquisition process and the expected length of the acquisition. In one example, the medical data acquisition information may be provided as video sequences, sound and voice in a headset, an immersive experience that describes the perception of being in the acquisition environment e.g., as it will be in the MR InBore experience or as a multisensory rhythmically synchronous stimulation. The rhythmical synchronous multisensory stimulation may, for example, include auditory, visual, vestibular and tactile sensory stimulation which in each sensory domain changes rhythmically in a synchronous way. Such multisensory stimulation may increase attentional focus and concentration of the user.

In one example, the interactive programs may perform at least part of the training steps of the preparation phase. For example, the number of interactive programs may be equal to the number N of training steps, e.g., each interactive program may perform a respective training step. Alternatively, the number of interactive programs may be smaller than the number N of training steps e.g., one interactive program may perform more than one training step or only part of the training steps are covered by the interactive programs. Alternatively, the number of interactive programs may be higher than the number N of training steps e.g., more than one interactive program may perform one training step or one interactive program may perform only part of a training step.

The user systems may be placed in respective locations in a building where the medical system is located. The location of the medical system may, for example, be an exam room. According to one example, the user system comprises any one of: headset, portable device, a TV, a computer system, or any system that can execute the interactive program. The headset may, for example, instruct the patient to follow a predefined breath-hold during an MRI acquisition e.g., using simple breathing commands. The headset may, for example, play breathing or breath-hold guidance, as will be needed in the MR scanner. The headset may, for example, accustom the user to the MR noise, by guiding the user through an increasing sequence of MR noises, both in noise, in duration and noise type. This may be personalized to the sequence scheduled for that user. The user system may, for example, comprise a goggle for virtual reality presentation or augmented reality through see-through glass.

The user systems may, for example, be configured to communicate with the medical system using one or more networks such as a local area network (LAN), a general wide area network (WAN), and/or a public network (e.g., the Internet). Additionally, or alternatively, the user systems may be configured to communicate with each other using one or more networks such as a local area network (LAN), a general wide area network (WAN), and/or a public network (e.g., the Internet). Additionally, or alternatively, the user systems may be configured to communicate with an acquisition scheduler system using one or more networks such as a local area network (LAN), a general wide area network (WAN), and/or a public network (e.g., the Internet). The acquisition scheduler system may be a computer system. The acquisition scheduler system may be configured to perform at least part of the present method. For example, the acquisition scheduler system may receive the user profile, determine the acquisition scheme and control the guiding of the user and the subsequent initiation of the acquisition process.

For example, after determining the acquisition scheme, the acquisition scheduler system may prompt the user to follow the route and the timeline by providing the route and/or timeline to the user. In response, the user may follow the route and assist to the interactive programs of the acquisition scheme. The acquisition scheduler system may control the selected user systems of the acquisition scheme, e.g., by sending control signals to them, in order to execute the interactive programs in accordance with the time intervals. In one example, the acquisition scheduler system may automatically detect that the user has completed all interactive programs e.g., by configuring every user system or just the last user system of the acquisition scheme to send a message indicating that the user has followed or attended the interactive program of the user system that sent the message. Using the message(s), the acquisition scheduler system may detect whether the user has completed all interactive programs. Alternatively, the user may be requested to provide a confirmation (e.g., as an input data) to the last user system of the acquisition scheme or to the medical system in order to indicate that all interactive programs have been followed by the user. This confirmation may automatically be sent to the acquisition scheduler system by the user system or by the medical system. After all interactive programs have been followed by the user, the state of the user may change to a desired value e.g., the state of the user may indicate a number of training steps higher than the threshold.

After determining that the user has followed the route or that all interactive programs have been followed by the user, the acquisition scheduler system may initiate the acquisition process by the medical system. Alternatively, after determining that the user has followed the route or that all interactive programs have been followed by the user, the acquisition scheduler system may further determine whether the current state of the user is in a desired value and if so, the acquisition scheduler system may initiate the acquisition process by the medical system. For example, the acquisition scheduler system may send a control signal to the medical system indicating that the acquisition process may be started for the user. For example, the acquisition scheduler system may control the medical system to display or provide the interactive programs followed by the user and/or display the current state of the user. These may be used for example to shorten the preparation phase and/or acquisition phase.

According to one example, the acquisition scheme comprises the timeline, the route and the interactive programs. The guiding of the user further comprises: automatically detecting at a certain point of the route an event that requires a change of the acquisition scheme. The acquisition scheme may be adapted according to the event. The user may be guided from the certain point to the medical system using the adapted scheme. The certain point of the route may include the last point of the route after completing the interactive programs and before entering the exam room. The user may have to be guided to follow another route starting from that certain point.

This example may enable an up-to-date acquisition scheme which may further improve the acquisition efficiency compared to a static acquisition scheme that may be suitable for specific situations only. Indeed, the patient route may be highly adaptive. For example, the user systems may be equipped with a feature to locate the user. Using the user location, the route may be adapted, adding or removing user systems to achieve minimal process time for the user and minimal idle time for the medical system. For example, if an event represents a specific user's input to a current interactive program, the subsequent interactive program(s) may be updated and the route may be updated as well to remove or add user systems better fitting the updated state. For example, the multisensory rhythmically synchronous stimulation may be adapted (e.g., based upon longitudinal data) in a straightforward way to adapt to the updated user profile of the user. For example, the rhythm or frequency (or multiples of it) of the breathing pace can be a basis for the rhythm of the multisensory stimulation.

According to one example, the detected event may comprise any one of:
a change in values of one or more attributes of the user profile; at least one of the interactive programs is started and/or ended outside the respective time interval and the state of the user at the end of the route is not in a desired value. The present subject matter may provide detection means to detect the event. The detection means may be the user system, a camera, a wristband measuring skin conductance, a blood pressure measuring means, electroencephalography (EEG) measuring headset that is attached to the user, movement detection means or other means. The user system on which the interactive program runs may determine whether the interactive program is executed within the required time interval. The camera may be used to detect the heart rate of the user via skin color changes, pupil diameter, and/or the speed of eye movement. The wristband measuring skin conductance may estimate current stress and predict upcoming stress. The blood pressure may be measured by blood pressure measuring means. The EEG measuring headset may be used to quantify which interactive program increases the alpha activity the most. Based on the user interaction with certain interactive programs, a further acquisition scheme may be used. The movement detection means may be used for detecting the movements of different body parts of the user. The movement detection means may include wearable sensors and environmental sensors such as optical sensors and wifi sensors. The detection means may be configured to detect events using speech and facial features from the user. The detection means may be configured to communicate the measurements results to the acquisition scheduler system using one or more networks such as a local area network (LAN), a general wide area network (WAN), and/or a public network (e.g., the Internet). Based on the received measurements, the acquisition scheduler system may determine whether the event occurred or not.

According to one example, the acquisition scheme may be adapted by adapting at least one of: the timeline, the route, or the interactive programs. For example, upon detecting the event by the acquisition scheduler system, the acquisition scheduler system may automatically adapt the timeline and/or the route and/or the interactive programs. The adaptation of the timeline may, for example, comprise changing the time interval of one or more interactive programs and/or excluding one or more interactive programs and/or adding one or more interactive programs and/or adapting one or more interactive programs. The adaptation of the interactive program may comprise changing instructions of the interactive program to provide another training feature. The training feature may, for example, be provided as a means to collect specific (user) data from the user without necessarily providing any stimulation. The adaptation of the route may be performed even if the timeline and the interactive programs are not changed by, for example, changing the path between two or more user systems.

According to one example, the execution of the interactive program comprises:
determining by the interactive program a user confirmation of completion of the interactive program, wherein the acquisition process is performed in case the number of confirmations is higher than or equal to a threshold. The number of confirmations may indicate the number of training steps followed by the user. For example, part or all interactive programs may be configured to request the confirmation of the user by, for example, pushing by the user a button on the user system or by providing a voice message by the user or by a specific gesture of the user. The confirmation may indicate that the user has succeeded the training step of the interactive program. This training step may be skipped in the preparation phase. This example may enable a controlled acquisition of medical data. For example, if the number of confirmations is smaller than a threshold, the user may not be allowed to enter the exam room and the method may be repeated for the user by updating the user profile based on results sent by the detection means, and updating the acquisition scheme based on the updated user profile, so the user may be guided again through the updated route.

According to one example, the guiding of the user is performed by executing a mobile application in a mobile device held by the user. For example, the acquisition scheduler system may automatically control the mobile application to display on the mobile device the route that the user follows. The timeline may further be displayed on the mobile device. The locations of the user systems may also be indicated on the display of the mobile device in association with the respective time intervals. The current location of the user on the route may also be indicated on the display. This example may enable a synchronized human-machine interaction process (e.g., the user can be in the right place at the right time) and a time efficient (e.g., faster) execution of the present method as it may provide precise information to the user about the route and the acquisition scheme. In one example, the mobile device may be used as a user system that may execute a respective interactive program in a specific time interval.

In another example, the route and timeline may be provided by voice messages instructing the user along the route. In another example, the route and timeline may be displayed in the building to help the user follows the route. For example, the route may be provided as a printed-out floor plan with a route and notes/explanations for the user systems of the user. In another example, the route may be provided using a digital display based on a (detected) location of the user. The route may also be presented as a combination of printed floor plan and audio-guidance.

According to one example, the method further comprises: pipelining the acquisition of the user with the acquisition of another user comprising: performing the acquisition scheme determination for the further user using a user profile of the further user such that the acquisition process is performed sequentially for the two users, and performing concurrently the guiding step for the two users. This may enable to acquire sequentially the medical data from the two users with a minimum predefined time delay. In one example, the acquisition schemes of two or more users may be determined by the acquisition scheduler system concurrently or sequentially.

This example may enable to acquire medical data from a high number of users with improved control of the acquisition process time. The route planning of the acquisition scheme of one user might take other user's location into account, e.g., to match users with similar states at the same user systems. For example, the acquisition scheduler system may identify common instructions or medical data information required by the users such that several users may follow one or more same interactive programs in respective user system.

According to one example, the user profile comprises values of multiple attributes that define the state of the user, wherein the attributes represent two or more of: user age; heart rate patterns of the user, skin conductance of the user, skin temperature of the user, respiration patterns of the user, movement patterns of the user, posture of the user or gait of the user; number of training steps followed by the user; number of medical imaging data acquisitions from the user; number of successful medical imaging data acquisitions of the user; number of unsuccessful medical imaging data acquisitions of the user. In one example, the number of training steps, the number of medical imaging data acquisitions from the user, the number of successful medical imaging data acquisitions of the user or the number of unsuccessful medical imaging data acquisitions of the user may define the training status of the user. The medical imaging data acquisitions may include MRI acquisitions or CT acquisitions. The previous medical imaging data acquisitions may improve the information level or experience of the user e.g., the higher the number of previous acquisitions the shorter the route to reach the exam room may be. The measured heart rate patterns of the user, eye movement of the user, facial expression of the user, skin conductance of the user, skin temperature of the user and posture, or gait of the user may, for example, indicate the anxiety level or fear level of the user.

According to one example, the user profile comprises attribute values which represent user state parameters of the user. The method further comprises: monitoring the user state parameters of the user during the guiding using at least one or more of the user systems, wherein detecting the event comprises detecting a change in the user state parameter of the user compared to the user state parameter in the user profile or compared to a target value of the user state parameter.

According to one example, the user profile comprises values of multiple attributes, wherein attribute values represent user state parameters of the user. The method further comprises creating the user profile by at least: receiving inputs as user inputs from the user and/or as measurement results from sensors configured to measure user state parameters of the user; wherein the measurements are performed before and/or during the guiding preferably using at least one or more of the user systems, determining values of the attributes using the inputs, creating a data structure comprising the attribute values, and providing the data structure as the user profile. For the user state parameters that are measured during the guidance step, the sensors may, for example, be part of the detection means described above. In one example, the acquisition scheduler system may comprise an interactive terminal and an AI engine such that user inputs may be obtained using the interactive terminal that asks questions with the AI engine or guides the user through a questionnaire to determine the attribute values. The questions or questionnaire may be adapted to the patient's answers to previous questions. Alternatively, the mobile application may be used to acquire the user inputs using the questions or questionnaire. Using the mobile application may be advantageous as the user profile may be created at least partially before the user comes to the hospital. Another example to provide the user profile is an interactive process with a human, where a greeting person from the hospital staff, e.g., a nurse, talks to the user and inputs the learned state of the user into the acquisition scheduler system.

According to one example, the user profile is received from a central server or from the mobile device of the user. For example, the acquisition scheduler system may receive the user profile from the central server or from the mobile device of the user. This may be performed automatically. Alternatively, the acquisition scheduler system may request from the central server or from the mobile device of the user the user profile and in response to the request may receive the user profile. This example may save processing resources that would otherwise be required by the acquisition scheduler system to create the user profile a new using user inputs. For example, after launching the mobile application on the mobile device, the mobile application may log into the acquisition scheduler system, e.g., via a QR-code, to provide the user profile and/or load the determined route and use the mobile application for navigation.

According to one example, the initially determined acquisition scheme may be considered as a partial schedule. In this case, the method further comprises a continuous triaging at different user systems of the initial acquisition scheme and depending on the state of the user, the acquisition scheme may be updated e.g., by defining next user systems. For example, at the entrance only user systems A and B may be suggested by the initial acquisition scheme. During the visit of user system B, the user state may be re-evaluated and then the next user systems for the user to visit may be suggested, which can be user systems C, D and E.

In another aspect the invention relates to a computer program comprising machine executable instructions for acquisition of medical imaging data using an acquisition process from a user, wherein a duration of the acquisition process depends on a state of the user to be imaged, wherein execution of the machine executable instructions causes a computational system to:

-   receive a user profile indicating an initial state of the user, wherein the duration of the acquisition process from the user in the initial state is an acquisition time period;
-   determine an acquisition scheme comprising:

    -   determining a timeline representing a chronological sequence of interactive programs and associated time intervals, the interactive programs being configured to change the initial state of the user;
-   selecting user systems that are configured to perform the interactive programs,
-   using the locations of the user systems to determine a route from a current location of the user to the location of the medical system;

-   provide the route and the timeline for guidance of the user through the route, the providing comprising controlling the selected user systems to execute the interactive programs in accordance with the time intervals of the timeline; thereby changing the initial state of the user; and thereafter
-   initiate the acquisition process from the user for reducing the acquisition time period.

In another aspect the invention relates to an acquisition scheduler system for acquisition of medical imaging data using an acquisition process from a user, wherein a duration of the acquisition process depends on a state of the user to be imaged, the acquisition scheduler system comprising: a memory storing machine executable instructions; and a computational system, wherein execution of the machine executable instructions causes the computational system to:

-   receive a user profile indicating an initial state of the user, wherein the duration of the acquisition process from the user in the initial state is an acquisition time period;
-   determine an acquisition scheme by at least:

    -   determining a timeline representing a chronological sequence of interactive programs and associated time intervals, the interactive programs being configured to change the initial state of the user;
    -   selecting user systems that are configured to perform the interactive programs,
    -   using the locations of the user systems to determine a route from a current location of the user to the location of the medical system;

-   provide the route and the timeline for guidance of the user through the route, the providing comprising controlling the selected user systems to execute the interactive programs in accordance with the time intervals of the timeline; thereby changing the initial state of the user; and thereafter
-   initiate the acquisition process from the user for reducing the acquisition time period.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance image or MR image is defined herein as being the reconstructed two- or three-dimensional visualization of anatomic data contained within the k-space data. This visualization can be performed using a computer.

BRIEF DESCRIPTION OF THE DRAWINGS

In the following preferred embodiments of the invention will be described, by way of example only, and with reference to the drawings in which:

Fig. 1 illustrates an example of an acquisition system;
Fig. 2 illustrates an example of an acquisition scheduler system;
Fig. 3 shows a flowchart of a method of acquiring medical data;
Fig. 4 shows a flowchart of a method of acquiring medical data;

Fig. 5 shows a flowchart of a method of acquiring medical data.

DETAILED DESCRIPTION OF EMBODIMENTS

Like numbered elements in these figures are either equivalent elements or perform the same function. Elements which have been discussed previously will not necessarily be discussed in later figures if the function is equivalent.

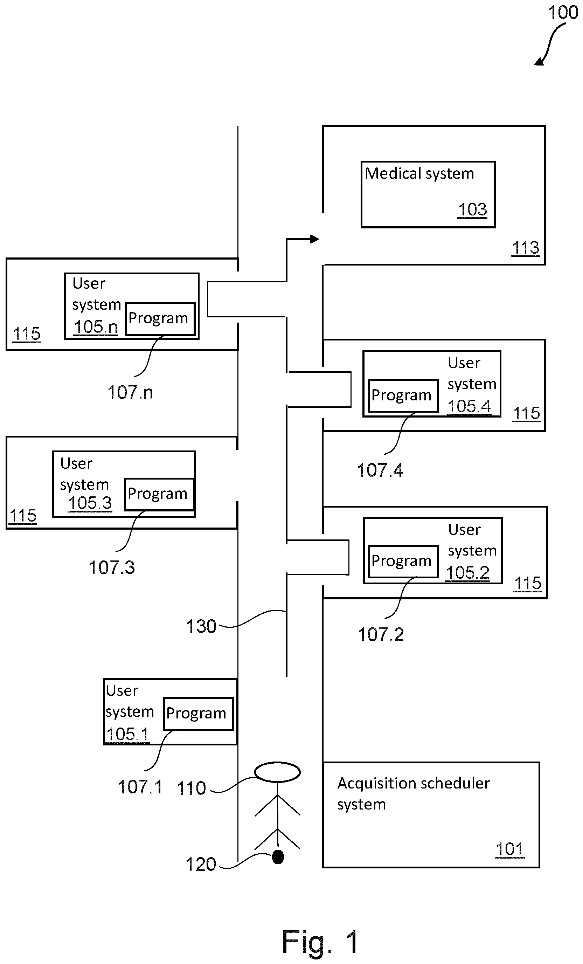

Fig. 1 illustrates an example of an acquisition system 100 in accordance with an example of the present subject matter. The acquisition system 100 comprises an acquisition scheduler system 101, a medical system 103 and multiple user systems 105.1 through 105.n. The medical system 103 may be located in a room (e.g., exam room) 113 of a building (e.g., hospital's building). The user system may be located in a room (e.g., experience zone) 115 or may be placed (such as user system 105.1) in a wall of a corridor of the building or in a space in the building or it can be an experience triggered by that specific location but presented via wearable means, for instance via the headphones, glasses or virtual reality headsets. The user systems 105.1 through 105.n may be configured to execute interactive programs 107.1 through 107.n respectively. The acquisition scheduler system 101 may be configured to receive inputs from at least one user 110. The current location 120 of the user 110 may for example be near the acquisition scheduler system 101. The user 110 may for example follow the route 130 which is defined by the acquisition scheduler system 101 in order to reach the exam room 113.

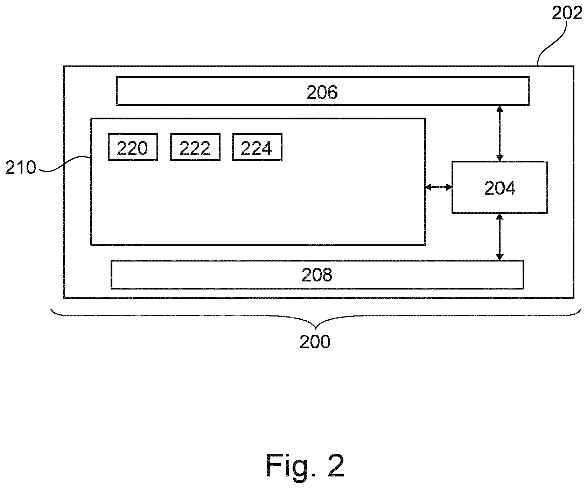

Fig. 2 illustrates an example of an acquisition scheduler system 200 in accordance with an example of the present subject matter. In this example the acquisition scheduler system 200 comprises a computer 202 with a computational system 204. The computer 202 is intended to represent one or more computers or computer systems. The computational system 204 is intended to represent one or more computational systems or computational cores. The computer 202 is further shown as containing an optional hardware interface 206 which is connected to the computational system 204. If other components of the acquisition scheduler system 200 are present or included such as a magnetic resonance imaging system, then the hardware interface 206 could be used to exchange data and commands with these other components. The acquisition scheduler system 200 is further shown as comprising an optional user interface 208 which may provide various means for relaying data and receiving data and commands from an operator.

The acquisition scheduler system 200 is further shown as comprising a memory 210 that is connected to the computational system 204. The memory 210 is intended to represent various types of memory which may be accessible to the computational system 204. The memory 210 is shown as containing machine-executable instructions 220. The machine-executable instructions 220 are instructions which enable the computational system 204 to perform various control, data processing, and image processing tasks. The memory 210 is further shown as containing one or more user profiles 222 that have been received and/or created by the acquisition scheduler system 200. The user profile 222 may, for example, be used to determine an acquisition scheme 224 for a user in accordance with the present subject matter.

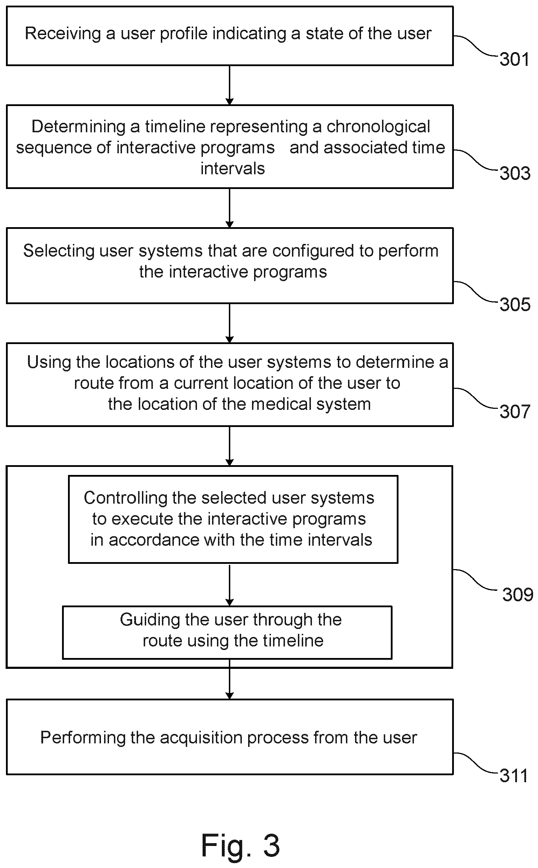

Fig. 3 is a flowchart of a method for acquiring medical data in accordance with an example of the present subject matter. For the purpose of explanation, the method described in Fig. 3 may be implemented in the system illustrated in Fig. 1, but is not limited to this implementation. The method may, for example, be performed by the acquisition scheduler system 101.

A user profile may be received in step 301. The user profile indicates an initial state of a user (e.g., patient). The duration of the acquisition process from the user in the initial state may be a first acquisition time period. An acquisition scheme may be determined in steps 303 to 307. In step 303, a timeline may be determined. The timeline represents a chronological sequence of interactive programs and associated time intervals. The interactive programs may be configured to change the initial state of the user. User systems that are configured to perform the interactive programs may be selected in step 305. The locations of the user systems may be used in step 307 to determine a route from a current location of the user to the location of the medical system. The timeline and route may be provided in step 309 to the user in order that the user follows the route and attend the interactive programs of the acquisition scheme. The provision in step 309 may comprise the control of the selected user systems to execute the interactive programs in accordance with the time intervals of the timeline. This may enable to change the initial state of the user. After completing the route, the acquisition process from the user may be triggered or initiated in step 311. The acquisition process may be performed using the medical system.

In one optional implementation of the method of Fig. 3, after executing step 309, and before performing step 311 it may be determined whether the state of the user has a target value. In case the state of the user has the target value, step 311 may be performed. In case the state of the user does not have the target value, steps 303 to 309 may be repeated. The repetition may be performed based on the same user profile of the last iteration or based on an updated user profile. The updated user profile may be obtained by updating the user profile of the last iteration using data obtained (e.g., by the detection means) during the guidance of the user in the last iteration. The repetition may be performed until a stopping criterion is fulfilled. The stopping criterion may, for example, require that the state of the user has the target value or that a maximum number of iterations is reached.

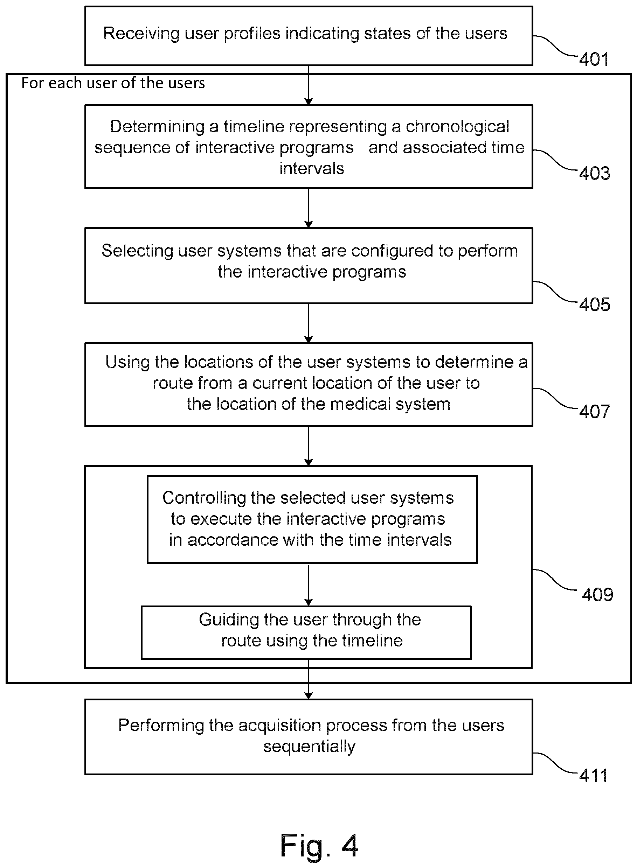

Fig. 4 is a flowchart of a method for acquiring medical data in accordance with an example of the present subject matter. For the purpose of explanation, the method described in Fig. 4 may be implemented in the system illustrated in Fig. 1, but is not limited to this implementation. The method may, for example, be performed by the acquisition scheduler system 101.

User profiles may be received in step 401. The user profiles indicate the initial states of the users (e.g., patients). For each user of the users, steps 403 to 409 may be performed. The duration of the acquisition process from the user in the initial state may be a first acquisition time period. An acquisition scheme may be determined in steps 403 to 407. In step 403, a timeline may be determined. The timeline represents a chronological sequence of interactive programs and associated time intervals. The interactive programs may be configured to change the initial state of the user. User systems that are configured to perform the interactive programs may be selected in step 405. The locations of the user systems may be used in step 407 to determine a route from a current location of the user to the location of the medical system. The timeline and route may be provided in step 409 to the user in order that the user follows the route. The provision in step 409 may comprise the control of the selected user systems to execute the interactive programs in accordance with the time intervals of the timeline. This may enable to change the initial state of the user. After completing the route by each user, the acquisition process from the user may be triggered or initiated in step 411 following a sequential order.

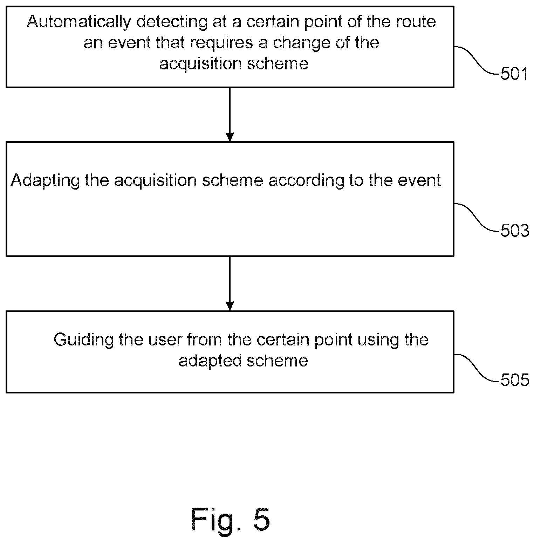

Fig. 5 is a flowchart of a method for acquiring medical data in accordance with an example of the present subject matter. For the purpose of explanation, the method described in Fig. 5 may be implemented in the system illustrated in Fig. 1, but is not limited to this implementation. The method may, for example, be performed by the acquisition scheduler system 101. The method of Fig. 5 may, for example, be performed during the guiding of the user e.g., during step 309 or step 409.

An event may automatically be detected at a certain point of the route in step 501. The event may require a change of the acquisition scheme (e.g., which is determined in the method of Fig. 3 or Fig. 4). The acquisition scheme may be adapted in step 503 according to the event. The user may be guided in step 505 from the certain point to the medical system using the adapted scheme. This may enable an up-to-date acquisition scheme which may further improve the acquisition efficiency compared to a static acquisition scheme that may be suitable for specific situations only.

**Claims**

1. A method for acquisition of medical imaging data using an acquisition process from a user, wherein a duration of the acquisition process depends on a state of the user to be imaged; the method comprising:

   - providing a medical system and multiple user systems, the systems being distributed according to predefined locations;
   - receiving a user profile indicating an initial state of the user, wherein the duration of the acquisition process from the user in the initial state is an acquisition time period;
   - determining an acquisition scheme comprising:

      - determining a timeline representing a chronological sequence of interactive programs and associated time intervals, the interactive programs being configured to change the initial state of the user;
      - selecting user systems that are configured to perform the interactive programs,
      - using the locations of the user systems to determine a route from a current location of the user to the location of the medical system;

   - providing the route and the timeline for enabling guidance of the user through the route, thereby changing the initial state of the user, the providing comprising controlling the selected user systems to execute the interactive programs in accordance with the time intervals of the timeline; and thereafter
   - initiating the acquisition process from the user for reducing the acquisition time period.

2. The method of claim 1, the acquisition scheme comprising the timeline, the route and the interactive programs, the guiding of the user further comprising:

   automatically detecting at a certain point of the route an event that requires a change of the acquisition scheme; and adapting the acquisition scheme according to the event; guiding the user from the certain point using the adapted scheme.

3. The method of claim 2, the event comprising any one of:

   a change in values of one or more attributes of the user profile; and at least one of the interactive programs is started and/or ended outside the respective time interval.

4. The method of claim 2 or 3, the user profile comprising attribute values which represent user state parameters of the user, the method further comprising:

monitoring the user state parameters of the user during the guiding using at least one or more of the user systems, wherein detecting the event comprises detecting a change in the user state parameter of the user compared to the user state parameter in the user profile.

5. The method of any of the preceding claims 2 to 4, wherein adapting the acquisition scheme comprises adapting at least one of: the timeline, the route, or the interactive programs.

6. The method of any of the preceding claims, wherein the execution of the interactive program comprises:

   determining by the interactive program a user confirmation of completion of the interactive program;
   wherein the acquisition process is performed in case the number of confirmations is higher than or equal to a threshold.

7. The method of any of the preceding claims, the user profile comprising values of multiple attributes, wherein attribute values represent user state parameters of the user, the method further comprising creating the user profile comprising:

   receiving inputs as user inputs from the user and/or as measurement results from sensors configured to measure user state parameters of the user; wherein the measurements are performed before and/or during the guiding preferably using at least one or more of the user systems;
   determining values of the attributes using the inputs;
   creating a data structure comprising the attribute values;
   providing the data structure as the user profile.

8. The method of any of the preceding claims 1 to 6, further comprising receiving the user profile from a central sever.

9. The method of any of the preceding claims, further comprising: pipelining the acquisition of the user with the acquisition of another user comprising:

   performing the acquisition scheme determination for the other user using a user profile of the other user such that the acquisition process is performed sequentially for the two users;
   wherein the guidance for the two users is performed concurrently.

10. The method of any of the preceding claims, the user profile comprising values of multiple attributes that define the state of the user, wherein the attributes representing two or more of:

   a. user age;
   b. user state parameters of the user; the user state parameters comprising at least one of: heart rate patterns of the user, skin conductance of the user, skin temperature of the user, respiration patterns of the user, eye and eyelids movements of the user, pupil size of the user, speech or sounds generated by user, facial expressions of the user, brainwave energy and frequencies of the user, movement patterns of the user, posture of the user or gait of the user;
   c. training steps followed by the user;
   d. number of medical imaging data acquisitions from the user;
   e. number of successful medical imaging data acquisitions of the user;
   f. number of unsuccessful medical imaging data acquisitions of the user.

11. The method of any of the preceding claims, the user system comprising any one of: headset, portable device, a TV, a computer system, or any system that can execute the interactive program.

12. The method of any of the preceding claims, wherein guiding the user is performed by executing a mobile application in a mobile device held by the user.

13. A computer program comprising machine executable instructions for acquisition of medical imaging data using an acquisition process from a user, wherein a duration of the acquisition process depends on a state of the user to be imaged, wherein execution of the machine executable instructions causes a computational system to:

   - receive a user profile indicating an initial state of the user, wherein the duration of the acquisition process from the user in the initial state is an acquisition time period;
   - determine an acquisition scheme comprising:

      - determining a timeline representing a chronological sequence of interactive programs and associated time intervals, the interactive programs being configured to change the initial state of the user;
      - selecting user systems that are configured to perform the interactive programs,
      - using the locations of the user systems to determine a route from a current location of the user to the location of the medical system;

   - provide the route and the timeline for guidance of the user through the route, the providing comprising controlling the selected user systems to execute the interactive programs in accordance with the time intervals of the timeline; thereby changing the initial state of the user; and thereafter
- initiate the acquisition process from the user for reducing the acquisition time period.

14. A system for acquisition of medical imaging data using an acquisition process from a user, wherein a duration of the acquisition process depends on a state of the user to be imaged, the system comprising: a memory storing machine executable instructions; and a computational system, wherein execution of the machine executable instructions causes the computational system to:

> - receive a user profile indicating an initial state of the user, wherein the duration of the acquisition process from the user in the initial state is an acquisition time period;
> - determine an acquisition scheme by at least:

>> - determining a timeline representing a chronological sequence of interactive programs and associated time intervals, the interactive programs being configured to change the initial state of the user;
>> - selecting user systems that are configured to perform the interactive programs,
>> - using the locations of the user systems to determine a route from a current location of the user to the location of the medical system;

> - provide the route and the timeline for guidance of the user through the route, the providing comprising controlling the selected user systems to execute the interactive programs in accordance with the time intervals of the timeline; thereby changing the initial state of the user; and thereafter
> - initiate the acquisition process from the user for reducing the acquisition time period.

15. The system of claim 14, comprising the medical system and/or the user systems, the medical system being a magnetic resonance imaging, MRI, system and the medical imaging data being MRI data.

Fig. 1

Fig. 2

Receiving a user profile indicating a state of the user ⎯301

Determining a timeline representing a chronological sequence of interactive programs and associated time intervals ⎯303

Selecting user systems that are configured to perform the interactive programs ⎯305

Using the locations of the user systems to determine a route from a current location of the user to the location of the medical system ⎯307

Controlling the selected user systems to execute the interactive programs in accordance with the time intervals

Guiding the user through the route using the timeline

⎯309

Performing the acquisition process from the user ⎯311

Fig. 3

Receiving user profiles indicating states of the users — 401

For each user of the users

Determining a timeline representing a chronological sequence of interactive programs and associated time intervals — 403

Selecting user systems that are configured to perform the interactive programs — 405

Using the locations of the user systems to determine a route from a current location of the user to the location of the medical system — 407

Controlling the selected user systems to execute the interactive programs in accordance with the time intervals

Guiding the user through the route using the timeline — 409

Performing the acquisition process from the users sequentially — 411

Fig. 4

Automatically detecting at a certain point of the route an event that requires a change of the acquisition scheme ⁓501

Adapting the acquisition scheme according to the event ⁓503

Guiding the user from the certain point using the adapted scheme ⁓505

# Fig. 5

Europäisches
Patentamt

European
Patent Office

Office européen
des brevets

# EUROPEAN SEARCH REPORT

**Application Number**

EP 23 18 0247

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/295985 A1 (PROKLE MICHAEL [US] ET AL) 23 September 2021 (2021-09-23) <br> * paragraph [0002] – paragraph [0004] * <br> * paragraph [0025] * <br> * paragraph [0029] * <br> * paragraph [0037] * <br> * paragraph [0031] – paragraph [0032] * <br> * paragraph [0051] * <br> * paragraph [0047] * <br> * figures 1,2 * | 1-15 | INV. <br> G16H40/20 <br> G16H50/70 <br> G16H10/60 <br> G16H20/00 <br> G16H30/20 |

-----

**TECHNICAL FIELDS SEARCHED (IPC)**

G16H

The present search report has been drawn up for all claims

| Place of search | Date of completion of the search | Examiner |
|---|---|---|
| Munich | 7 November 2023 | Gardiner, Alexander |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 18 0247

This annex lists the patent family members relating to the patent documents cited in the above-mentioned European search report.
The members are as contained in the European Patent Office EDP file on
The European Patent Office is in no way liable for these particulars which are merely given for the purpose of information.

07-11-2023

| Patent document cited in search report | Publication date | Patent family member(s) | Publication date |
|---|---|---|---|
| US 2021295985 A1 | 23-09-2021 | US 2021295985 A1<br>WO 2020016048 A1 | 23-09-2021<br>23-01-2020 |

EPO FORM P0459

For more details about this annex : see Official Journal of the European Patent Office, No. 12/82